(19) Europäisches Patentamt / European Patent Office / Office européen des brevets

(11) **EP 3 652 136 B1**

(12) **FASCICULE DE BREVET EUROPEEN**

(45) Date de publication et mention de la délivrance du brevet:
**25.01.2023 Bulletin 2023/04**

(21) Numéro de dépôt: **18735600.1**

(22) Date de dépôt: **10.07.2018**

(51) Classification Internationale des Brevets (IPC):
**C07C 1/24** *(2006.01)*    **C07C 11/09** *(2006.01)*

(52) Classification Coopérative des Brevets (CPC):
(C-Sets disponibles)
**C07C 1/24; C07C 11/09**    (Cont.)

(86) Numéro de dépôt international:
**PCT/EP2018/068613**

(87) Numéro de publication internationale:
**WO 2019/011892 (17.01.2019 Gazette 2019/03)**

(54) **PROCEDE DE DESHYDRATATION DES ALCOOLS EN OLEFINES COMPRENANT UNE ETAPE DE SELECTIVATION DU CATALYSEUR**

**VERFAHREN ZUR ENTWÄSSERUNG VON ALKOHOLEN ZUR GEWINNUNG VON OLEFINEN UNTER BETEILIGUNG EINES KATALYSATORSELEKTIVIERUNGSSCHRITTES**

**METHOD FOR DEHYDRATING ALCOHOLS TO OBTAIN OLEFINS, INVOLVING A STEP OF CATALYST SELECTIVATION**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.07.2017 FR 1756515**

(43) Date de publication de la demande:
**20.05.2020 Bulletin 2020/21**

(73) Titulaires:
• **IFP Energies nouvelles**
  **92852 Rueil-Malmaison (FR)**
• **Total Research & Technology Feluy**
  **7181 Seneffe (BE)**

(72) Inventeurs:
• **ARIBERT, Nicolas**
  **38430 Moirans (FR)**
• **COUPARD, Vincent**
  **69100 Villeurbanne (FR)**
• **MOUNIER, Julie**
  **38200 Vienne (FR)**
• **NESTERENKO, Nikolai**
  **14000 Nivelles (BE)**
• **THILLE, Christophe**
  **1982 Weerde (BE)**

(74) Mandataire: **IFP Energies nouvelles**
  **Département Propriété Industrielle**
  **Rond Point de l'échangeur de Solaize**
  **BP3**
  **69360 Solaize (FR)**

(56) Documents cités:
**WO-A1-2011/113834**

(52) Classification Coopérative des Brevets (CPC):
(Cont.)

C-Sets
**C07C 1/24, C07C 11/09**

**Description**

**DOMAINE TECHNIQUE DE L'INVENTION**

La présente invention concerne un procédé de déshydratation des alcools en oléfines. La charge du procédé peut être obtenue par des procédés chimiques ou par des procédés fermentaires. Ce procédé met en oeuvre un catalyseur mis en forme à base d'une zéolithe comprenant au moins une série de canaux dont l'ouverture est définie par un anneau à 8 atomes d'oxygène (8MR) et comprend une étape permettant d'améliorer la sélectivité du catalyseur.

Les alcènes obtenus, en particulier l'isobutène, le butène-1 et les butènes-2, présentent un intérêt important dans le domaine de l'industrie pétrochimique et de la synthèse organique.

**ART ANTÉRIEUR**

Le document EP 2348 005 décrit la déshydratation d'alcools contenant de 2 à 10 atomes de carbone en l'oléfine correspondante sur un catalyseur zéolithique FER de ratio atomique Si/Al inférieur à 100. La vitesse spatiale horaire massique (Weight Hourly Space Velocity selon la dénomination anglaise, ou WHSV) par rapport à l'alcool est d'au moins 4 $h^{-1}$ et la température de 320 à 600°C.

Le document WO 2011/113834 décrit la déshydratation et l'isomérisation squelettale simultanée de l'isobutanol en présence de catalyseurs silicates cristallins, à taille de canaux moyenne (10MR) déaluminisés ou non, modifiés au phosphore ou non, du groupe FER, MWW, EUO, MFS, ZSM-48, MTT, MFI, MEL ou TON ayant un ratio Si/Al supérieur à 10, tamis moléculaires silicoaluminophosphates du groupe AEL, ou silice-, zircone-, titane- ou fluor-alumine sur catalyseurs zéolithiques. La PPH (ratio du débit massique de charge sur la masse de catalyseur, correspondant à la WHSV) par rapport à l'alcool est d'au moins 1 $h^{-1}$ et la température de 200 à 600°C. La proportion maximale atteinte en n-butènes dans les butènes est de 58.4% à 375°C à forte PPH (12.6 $h^{-1}$) sur une zéolithe FER en poudre de Si/Al 33. Aucune notion de stabilité des performances en fonction du temps sous charge n'est évoquée dans ce document. Le seul autre catalyseur exemplifié est l'alumine gamma.

Les performances catalytiques (conversion et sélectivité vers la formation d'oléfines linéaires) diminuent en fonction de la durée d'opération du procédé du fait, principalement, de formation de coke pendant la réaction de déshydratation isomérisante. Afin de maintenir la cible en conversion, la température moyenne de réaction est augmentée, induisant des baisses de sélectivité. Ainsi, une étape de régénération est nécessaire lorsque soit la température maximale de réaction admissible est atteinte, soit la sélectivité en iso-oléfines dépasse une valeur fixée qui ne permet plus la rentabilité du procédé.

L'étape de régénération correspond principalement à une étape de combustion du coke formé sous air et en température décrite dans de nombreux documents. Par exemple, le document EP 3040125 décrit la régénération du catalyseur par combustion du coke formé en surface. Benito et al. (Ind.Eng.Chem.Res., 1996, 35, 2177-2182) décrivent des cycles de réaction-régénération, la régénération étant réalisée par combustion.

Mais cette étape engendre des surcoûts conséquents dans le procédé de fabrication des oléfines linéaires.

La sélectivité vers les oléfines linéaires décroissant souvent plus rapidement que la conversion vers des valeurs conduisant à opérer une régénération, c'est souvent ce facteur qui conduit à lancer une opération de régénération du catalyseur. Il a été découvert par la demanderesse un procédé particulier permettant d'améliorer la sélectivité et la stabilité du catalyseur. Cette amélioration permet de retarder le recours à une régénération lorsque celle-ci est conditionnée par le dépassement de la cible en sélectivité isobutène/butènes.

**OBJET ET INTÉRÊT DE L'INVENTION**

L'invention concerne un procédé de déshydratation des alcools en oléfines comprenant :

a) une étape réactionnelle alimentée par une charge comprenant au moins un monoalcool, primaire de formule R-$CH_2$-OH, dans lequel R est un radical alkyl non linéaire de formule générale $C_nH_{2n+1}$ où n est un entier compris entre 3 et 20, ladite étape réactionnelle étant opérée en phase gaz à une température moyenne pondérée comprise entre 250 et 400°C, à une pression comprise entre 0,2 MPa et 1 MPa et à une PPH comprise entre 1 et 18 $h^{-1}$ en présence d'un catalyseur comprenant une zéolithe comprenant au moins une série de canaux dont l'ouverture est définie par un anneau à 8 atomes d'oxygène (8 MR) produisant un effluent oléfinique ;
b) une étape de sélectivation comprenant, pendant une durée comprise entre 0,5 et 5 h, l'arrêt de l'alimentation et le maintien de la température et de la pression de ladite étape a) ;
c) la reprise de l'alimentation à l'issue de l'étape b) avec retour aux conditions de l'étape a) ; ladite étape de sélectivation étant mise en oeuvre lorsque la sélectivité en iso-oléfines n de ladite étape a) est supérieure à 25%, ou lorsque la conversion en alcools de ladite étape a) est inférieure à 99%.

Le procédé selon l'invention vise à produire principalement des oléfines linéaires. De manière étonnante, le procédé selon l'invention permet d'améliorer la sélectivité en butènes linéaires dans l'effluent oléfinique de manière significative sans modifier l'activité catalytique, c'est à dire en maintenant la conversion en alcools.

## DESCRIPTION DÉTAILLÉE DE L'INVENTION

### Etape réactionnelle a)

Le procédé selon l'invention comprend une étape a) réactionnelle alimentée par une charge comprenant au moins un alcool, ladite étape réactionnelle étant opérée en phase gaz à une température moyenne pondérée comprise entre 250 et 400°C, à une pression comprise entre 0,2 MPa et 1 MPa et à une PPH comprise entre 1 et 18 h$^{-1}$ en présence d'un catalyseur comprenant une zéolithe comprenant au moins une série de canaux dont l'ouverture est définie par un anneau à 8 atomes d'oxygène (8 MR) produisant un effluent oléfinique, ledit alcool étant monoalcool primaire de formule R-CH$_2$-OH, dans lequel R est un radical alkyl non linéaire de formule générale C$_n$H$_{2n+1}$ où n est un entier compris entre 3 et 20.

### Charge

La charge traitée dans le procédé selon l'invention est une charge comprenant au moins un alcool, c'est-à-dire au moins un monoalcool primaire de formule R-CH$_2$-OH, dans lequel R est un radical alkyl non linéaire de formule générale C$_n$H$_{2n+1}$ où n est un entier compris entre 3 et 20, de préférence entre 3 et 10, de manière préférée entre 3 et 5. Ladite étape réactionnelle a) est une étape dans laquelle l'alcool est déshydraté en oléfine, les alcools branchés étant avantageusement déshydratés en oléfines linéaires. Le procédé selon l'invention est avantageusement un procédé de déshydratation isomérisante.

De préférence, la charge comprend de 40 à 100% poids, préférentiellement de 70 à 100% poids, de manière avantageuse de 85 à 100% poids d'au moins un alcool, avantageusement d'au moins un monoalcool primaire tel que défini précédemment.

Parmi les monoalcools primaires utilisables dans le procédé selon l'invention, on peut citer l'isobutanol, le 2-methylbutan-1-ol, le 2,2-dimethylpropan-1-ol, le 2-methylpentan-1-ol, le 2,2-dimethylbutan-1-ol, le 2-ethylbutan-1-ol. Ladite charge peut comprendre un ou plusieurs monoalcool primaire.

Ledit monoalcool primaire est préférentiellement l'isobutanol ou le 2-methyl-1-butanol, pris seul ou en mélange. Très préférentiellement, ledit monoalcool primaire est l'isobutanol.

Ladite charge peut provenir de procédés chimiques ou biochimiques, par exemple fermentaires. En particulier, cette charge peut être issue de procédés de fermentation de biomasse lignocellulosique.

Ladite charge peut contenir de l'eau, avantageusement jusqu'à 60% poids d'eau, préférentiellement jusqu'à 70% poids, très avantageusement jusqu'à 15% poids. Elle peut également comprendre des impuretés de type minéral (telles que Na, Ca, P, Al, Si, K, SO$_4$) et de type organique (telles que du méthanol, de l'éthanol, du n-butanol, des aldéhydes, des cétones, et les acides correspondant, par exemple l'acide furanique, acétique, isobutyrique).

Ladite étape réactionnelle est opéré en phase gaz, à une température moyenne pondérée comprise entre 250 et 400°C, de préférence entre 250 et 375°C, à une pression comprise entre 0,2 MPa et 1 MPa, à une PPH comprise entre 1 et 18 h$^{-1}$, en présence d'un catalyseur comprenant une zéolithe comprenant au moins une série de canaux dont l'ouverture est définie par un anneau à 8 atomes d'oxygène (8 MR).

Par PPH, on entend « Poids par Poids par Heure », c'est-à-dire le débit massique de d'alcool dans la charge, en entrée de réacteur divisé par la masse de catalyseur dans ledit réacteur. Cette notion est également parfois désignée sous son acronyme anglais de WHSV, ou « Weight Hourly Space Velocity ».

Par température moyenne pondérée (noté TMP), on entend la moyenne de la température dans le lit catalytique, le lit étant l'ensemble des lits présents dans le réacteur, lits dans lesquels se déroule la réaction catalytique, calculée le long de l'axe de l'écoulement dans ledit lit. Soit un lit de longueur L et de surface S, le mélange réactif s'écoulant le long de l'axe longitudinal $x$ de ce lit, l'entrée dans le lit catalytique formant l'origine de l'axe ($x$=0), la température moyenne pondérée, noté TMP, s'exprime selon la formule suivante :

$$\text{TMP} = \frac{1}{L} \int_0^L T(x)dx$$

La réaction étant endothermique et le réacteur opérant soit en mode isotherme, soit en mode adiabatique, la température moyenne pondérée est représentative de la température de réaction. L'apport en calories est réalisé par tout moyen de chauffage connu de l'homme du métier.

La réaction se déroule dans un ou plusieurs réacteurs, chaque réacteur étant opéré dans des conditions identiques. La TMP de chacun des réacteurs est ajustée à une valeur comprise entre 250°C et 400°C, de préférence entre 250 et 375°C. Ainsi, dans la suite de l'exposé, le terme « le réacteur » désigne aussi bien le réacteur de cette étape lorsque celle-ci ne comprend qu'un réacteur, que chacun des réacteurs de cette étape, lorsque celle-ci comprend plus d'un réacteur.

Ledit catalyseur est disposé dans un ou plusieurs lits fixes, lesquels peuvent être opérés en écoulement ascendant, descendant ou radial.

Avant mise en contact avec la charge à traiter, le catalyseur est activé par tout moyen connu de l'homme du métier, par exemple par traitement thermique sous air.

**Catalyseur**

Conformément à l'invention, le catalyseur mis en oeuvre dans ladite étape réactionnelle a) comprend une zéolithe présentant au moins une série de canaux dont l'ouverture est définie par un anneau à 8 atomes d'oxygène (8MR) telle que définie dans la classification "Atlas of Zeolite Structure Types", Ch. Baerlocher, L. B. Mc Cusker, D.H. Oison, 6ème Edition, Elsevier, 2007, Elsevier".

Selon un mode de réalisation particulier, la zéolithe peut également avantageusement contenir au moins une série de canaux dont l'ouverture de pores est définie par un anneau contenant 10 atomes d'oxygène (10 MR).

Ladite zéolithe est avantageusement choisie parmi les zéolithes ayant des canaux 8 et 10MR telles que les zéolithes de type structural FER et MFS, prises seules ou en mélange. La zéolite est plus avantageusement choisie dans le groupe constitué par, pour le type FER, les zéolithes ferrierite, FU-9, ISI-6, NU-23, ZSM-35 et pour le type MFS, la zéolite ZSM-57, et leurs mélanges. Ladite zéolithe est très avantageusement de type FER, et de préférence la ferrierite. De préférence, ladite zéolite est constituée de ferrierite.

De façon préférée, ladite zéolite est une ferrierite de rapport molaire Si/Al de 8 à 70, de préférence 10 à 70, de préférence choisi entre 10 et 50, de manière préférée choisi entre 20 et 50.

Ledit catalyseur comprend également un liant.

La teneur en zéolite dans le catalyseur est de 55-90%pds, de préférence entre 60 et 80%pds.

Le liant est avantageusement choisi parmi un liant silicique, un $AlPO_4$, une argile, une zircone, un oxyde de Ti, du SiC. De façon très préférée, c'est un liant silicique.

La teneur en liant dans le catalyseur est comprise entre 10 et 45% pds, de préférence entre 20 et 40%. Le catalyseur peut éventuellement contenir des impuretés en faible quantité n'ayant pas d'effet technique sur la conversion /sélectivité du catalyseur. Ledit catalyseur peut être mis en forme par toutes les techniques connues de l'Homme du métier, par exemple sous forme de poudre, de billes, de pastilles, de granulés, ou d'extrudés (cylindres creux ou non, cylindres multilobés à 2, 3, 4 ou 5 lobes par exemple, cylindres torsadés), d'anneaux, etc..

Généralement, le catalyseur ne comprend pas de métaux. On comprend par cette expression « pas de métaux », qu'il n'y a pas de métaux ajoutés lors de la préparation. On comprend également qu'il peut y avoir des impuretés dans les liants et donc en faibles quantités. De manière préférée, il n'y a pas d'aluminium ou de fer dans la silice.

**Etape de sélectivation b)**

Le procédé selon l'invention comprend une étape b) de sélectivation du catalyseur comprenant, pendant une durée comprise entre 0,5 et 5 h, l'arrêt de l'alimentation et le maintien de la température et de la pression de ladite étape a), ladite étape de sélectivation étant mise en oeuvre lorsque la sélectivité en iso-oléfines n de ladite étape a) est supérieure à 25%, ou lorsque la conversion en alcools de ladite étape a) est inférieure à 99%.

Par sélectivité en iso-oléfines *n,* on entend le ratio massique d'iso-oléfines comprenant *n* atomes de carbone sur la totalité des oléfines comprenant n atomes de carbone présentes dans l'effluent oléfinique issu de l'étape a). La sélectivité en iso-oléfines n augmente progressivement lors de l'étape a) du procédé selon l'invention.

La conversion de l'alcool est calculée de la manière suivante :

$$Conversion_{alcool} = \left( 1 - \frac{\sum\limits_{alcools} m_{alcools\ sortie}}{\sum\limits_{alcools} m_{alcools\ entrée}} \right) \times 100$$

Plus spécifiquement, dans le cas où l'alcool est l'iso-butanol :

$$Conversion_{iC4OH} = \left(1 - \frac{m_{iC4OH\ sortie}}{m_{iC4OH\ entrée}}\right) \times 100$$

Les compositions en entrée et sortie de l'étape réactionnelle nécessaires au calcul des sélectivités et conversions sont déterminées par les méthodes bien connues de l'Homme du métier, par exemple par prise d'échantillon et analyse, en particulier par analyse par chromatographie en phase gaz, la prise d'échantillon pouvant être réalisée en continue ou ponctuellement, à des intervalles de temps permettant le suivi de l'évolution des valeurs de sélectivité et/ou de conversion, cet intervalle étant aisément déterminé par l'Homme du métier (par exemple toutes les minutes, toutes les 5 minutes, toutes les 10 minutes, toutes les demi-heure, toutes les heures).

La demanderesse a découvert que l'arrêt de l'alimentation réalisé conjointement au maintien de la température et de la pression de ladite étape a) pendant une durée comprise entre 0,5 et 5 h, avantageusement entre 0,5 et 3 h, très avantageusement entre 0,5 et 2 h permettait de réduire la sélectivité en iso-oléfines tout en maintenant au moins la conversion de l'alcool en oléfines une fois l'alimentation redémarrée à l'issue de la durée d'arrêt et les conditions de l'étape a) rétablies.

Le maintien de la température et de la pression peut être réalisé par tout moyen connu de l'Homme du métier. Par exemple, le maintien de la pression peut être réalisé en diminuant, voire arrêtant, la production d'effluent de ladite étape réactionnelle, ou bien en ajoutant un fluide inerte, tel que par exemple de l'azote.

De manière avantageuse, ladite étape b) de sélectivation comprend également l'arrêt de production d'effluent de ladite étape réactionnelle a).

Lorsque ladite étape b) est réalisée pendant une durée inférieure à 0,5 h, aucun effet n'est observé. A contrario, un arrêt prolongé au-delà de 5 h conduit à la formation de produits indésirables à la surface du catalyseur qui nuisent aux performances du procédé.

**Etape c) de reprise de l'alimentation**

Le procédé selon l'invention comprend une étape c) de reprise de l'alimentation à l'issue de l'étape b) avec retour aux conditions de l'étape a).

À l'issue de la durée d'arrêt de l'alimentation conformément à l'étape b) du procédé selon l'invention, l'alimentation est reprise afin de revenir aux conditions de l'étape a) dudit procédé. À l'issue de l'étape b), le procédé selon l'invention fonctionne donc conformément aux caractéristiques de l'étape a) du procédé selon l'invention, la sélectivité en iso-oléfine n ayant été diminuée.

**DESCRIPTION DES FIGURES**

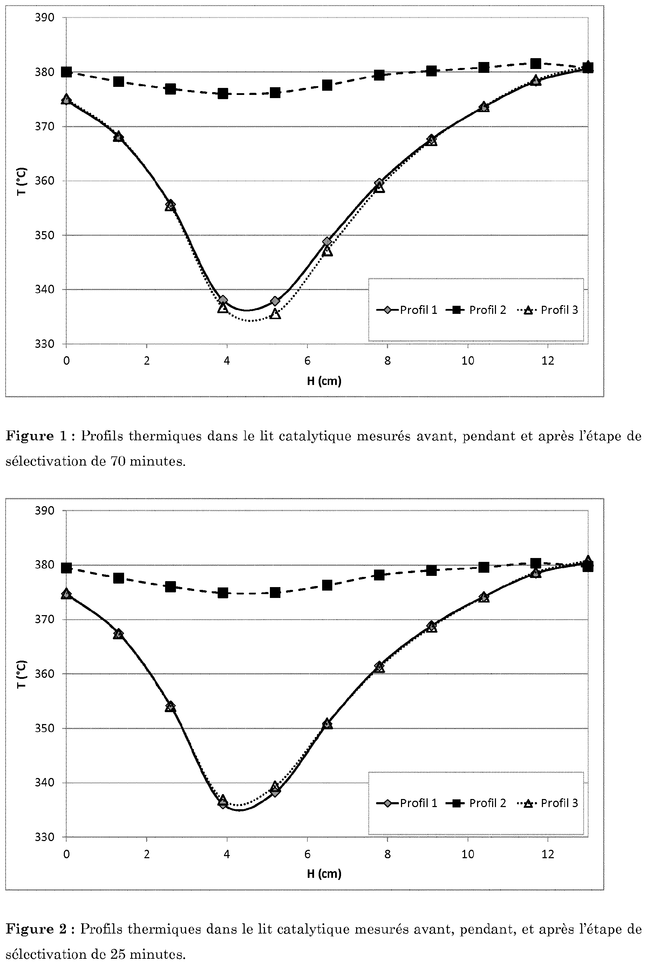

Les **figures 1 et 2** montrent les profils axiaux de température dans le réacteur avant l'étape réactionnelle a) (profil 1), pendant l'étape réactionnelle a) (profil 2) et après l'étape de sélectivation b) une fois reprise l'alimentation de la charge (profil 3).

**EXEMPLES**

Description de l'unité de test catalytique utilisé pour les exemples 1 et 2.

L'étape de déshydratation est réalisée sur une unité de test catalytique comprenant un lit fixe fonctionnant en mode « down flow », c'est-à-dire en écoulement descendant. Le catalyseur est chargé dans un réacteur inox 316L sur une hauteur de 13 cm. Le catalyseur est ensuite activé à 450°C sous 24 Nl/h d'air pendant un palier de deux heures après une montée en température de 50°C/h. La température est ensuite abaissée à la température de test sous 500 Nl/h d'azote afin d'éliminer l'air présent dans le système avant injection de la charge alcool.

La charge est vaporisée dans un four de préchauffe à 150-200°C en amont du réacteur puis injectée dans le réacteur catalytique. Les conditions opératoires sont les suivantes : température moyenne pondérée de 362°C, pph (poids de charge par poids de catalyseur par gramme) de 8,5 h$^{-1}$.

En sortie de réacteur, l'effluent total passe par deux séparateurs, l'un fonctionnant à la pression de réaction et l'autre à faible pression, afin de récupérer d'une part un effluent gaz riche en oléfines et une phase liquide riche en eau. L'analyse de l'effluent gazeux est effectuée sur un chromatographe en phase gazeuse en ligne équipée de deux colonnes. L'analyse de l'effluent liquide est réalisée sur un appareil délocalisé par chromatographie en phase gazeuse. Ces analyses permettent de déterminer la conversion de l'isobutanol, les sélectivités en différents produits et notamment la sélectivité en iso-butène et la fraction de butènes linéaires dans l'effluent de réaction, fraction que l'on cherche à maximiser. Les analyseurs permettent aussi de mesurer la sélectivité en produits secondaires tels que le propène ou les produits contenant 5 atomes de carbone ou plus.

**Conversion de l'isobutanol :**

Cet indicateur permet d'évaluer l'activité du catalyseur.

$$Conversion_{iC4OH} = \left(1 - \frac{m_{iC4OH\ sortie}}{m_{iC4OH\ entrée}}\right) \times 100$$

**Sélectivité en isobutène dans la coupe oléfinique en C4**

Afin de mesurer l'isomérisation de l'effluent oléfinique, un indicateur basé sur la quantité (en masse de carbone équivalent) de butènes linéaires formés a été défini.

$$Sélectivité_{isobutène} = \left(\frac{masse\ isobutène_{sortie}}{masse\ butènes_{sortie}}\right) \times 100$$

**Exemple 1 (conforme): procédure de 70 minutes, durée suffisante**

*Dans cet exemple, l'arrêt de l'alimentation est réalisé pendant 70 min, les autres conditions opératoires étant maintenues. La sélectivité en iso-butène est diminuée.*

Une charge comprenant 95% poids d'isobutanol et 5% poids d'eau est alimentée en entrée de réacteur avec une pph de 8,5 $h^{-1}$. L'étape réactionnelle est opérée à une pression de 0,9 MPa et une température moyenne pondérée (TMP) de 362°C.

Après 60 heures de de durée sous charge, la sélectivité en iso-butène dans l'effluent oléfinique atteint 23,9%.. L'alimentation en charge est interrompue, ainsi que le soutirage en effluent oléfinique. Les températures et pression sont maintenues dans l'étape réactionnelle.

L'arrêt momentané du débit de charge est réalisé pendant 70 minutes. Après reprise de l'alimentation de la charge dans les mêmes conditions qu'initialement (pph de 8,5 $h^{-1}$), la sélectivité en isobutène dans l'effluent oléfinique est de 19,3%, soit une différence de 4,6 points.

La conversion en isobutanol est supérieure à 99,95%, identique à la conversion avant arrêt.

La **figure 1** montre les profils axiaux de température dans le réacteur avant l'étape réactionnelle a) (profil 1), pendant l'étape réactionnelle a) (profil 2) et après l'étape de sélectivation b) une fois reprise l'alimentation de la charge et stabilisées les conditions conformément à l'étape a) (profil 3) après l'arrêt de l'alimentation pendant 70 min. En abscisse est représentée la hauteur totale du réacteur. L'axe des ordonnées représente la température dans le réacteur.

On observe sur la figure 1 que les profils thermiques avant et après l'étape de sélectivation (profil 1 et profil 3) sont sensiblement les mêmes. Ce gain n'est donc pas dû à une modification du profil thermique au sein de l'étape réactionnelle.

**Exemple 2 (non-conforme): procédure de 25 minutes, durée insuffisante**

*Dans cet exemple, l'arrêt de l'alimentation est réalisé pendant 25 min, les autres conditions opératoires étant maintenues. La sélectivité en iso-butène n'est pas modifiée.*

Une charge comprenant 95% poids d'isobutanol et 5% poids d'eau est alimentée en entrée de réacteur avec une pph de 8,5 $h^{-1}$. L'étape réactionnelle est opérée à une pression de 0,9 MPa et une TMP de 362°C.

Après 30 heures de de durée sous charge, la sélectivité en iso-butène dans l'effluent oléfinique atteint 24,0%. L'alimentation en charge est interrompue, ainsi que le soutirage en effluent oléfinique. Les températures et pression sont maintenues dans l'étape réactionnelle.

L'arrêt momentané du débit de charge est réalisé pendant 25 minutes. Après reprise de l'alimentation de la charge dans les mêmes conditions qu'initialement (pph de 8,5 $h^{-1}$), la sélectivité en isobutène dans l'effluent oléfinique est de 23.8%, soit une différence non significative de de 0,2 point située dans l'erreur de mesure.

La conversion en isobutanol est supérieure à 99,95%, identique à la conversion avant arrêt.

La **figure 2** montre les profils axiaux de température dans le réacteur avant l'étape réactionnelle a) (profil 1), pendant l'étape réactionnelle a) (profil 2) et après l'étape de sélectivation b) une fois reprise l'alimentation de la charge et stabilisées les conditions conformément à l'étape a) (profil 3) après l'arrêt de l'alimentation pendant 25 min. En abscisse est représentée la hauteur totale du réacteur. L'axe des ordonnées représente la température dans le réacteur.

On observe sur la figure 2 que les profils thermiques avant et après sélectivation sont sensiblement les mêmes. Ce gain n'est donc pas dû à une modification du profil thermique au sein de l'étape réactionnelle.

On observe par ailleurs sur la figure 2 que le profil de température pendant l'étape de sélectivation est similaire à celui obtenu lors de l'exemple 1. L'effet observé n'est donc pas dû à une simple remontée en température du lit pendant l'étape de sélectivation.

**Exemple 3 (non-conforme): procédure de 6 heures**

*Dans cet exemple, l'arrêt de l'alimentation est réalisé pendant 6 heures, les autres conditions opératoires étant maintenues.*

Une charge comprenant 95% poids d'isobutanol et 5% poids d'eau est alimentée en entrée de réacteur avec une pph de 8,5 $h^{-1}$. L'étape réactionnelle est opérée à une pression de 0,9 MPa et une TMP de 366°C.

Après 80 heures de durée sous charge, la sélectivité en iso-butène dans l'effluent oléfinique atteint 23,8% et la conversion en isobutanol est supérieure à 99,94%. L'alimentation en charge est interrompue, ainsi que le soutirage en effluent oléfinique. Les températures et pression sont maintenues dans l'étape réactionnelle.

L'arrêt momentané du débit de charge est réalisé pendant 6 heures.

Après reprise de l'alimentation de la charge dans les mêmes conditions qu'initialement (pph de 8,5 $h^{-1}$), la sélectivité en isobutène dans l'effluent oléfinique est de 19%.

La conversion en isobutanol est 97.53%.

Après un arrêt du débit de la charge pendant 6 heures, une diminution de la conversion en isobutanol de 99.94% à 97.53% est observée. Cette baisse de la conversion en isobutanol révèle une perte significative de l'activité du catalyseur.

**Revendications**

1. Procédé de déshydratation des alcools en oléfines comprenant :

   a) une étape réactionnelle alimentée par une charge comprenant au moins un monoalcool primaire de formule R-CH$_2$-OH, dans lequel R est un radical alkyl non linéaire de formule générale C$_n$H$_{2n+1}$ où n est un entier compris entre 3 et 20, ladite étape réactionnelle étant opérée en phase gaz à une température moyenne pondérée comprise entre 250 et 400°C, à une pression comprise entre 0,2 MPa et 1 MPa et à une PPH comprise entre 1 et 18 $h^{-1}$ en présence d'un catalyseur comprenant une zéolithe comprenant au moins une série de canaux dont l'ouverture est définie par un anneau à 8 atomes d'oxygène (8 MR) produisant un effluent oléfinique ;
   b) une étape de sélectivation du catalyseur comprenant, pendant une durée comprise entre 0,5 et 5 h, l'arrêt de l'alimentation et le maintien de la température et de la pression de ladite étape a) ;
   c) la reprise de l'alimentation à l'issue de l'étape b) avec retour aux conditions de l'étape a) ; ladite étape de sélectivation étant mise en oeuvre lorsque la sélectivité en iso-oléfines *n* de ladite étape a) est supérieure à 25%, ou lorsque la conversion en alcools de ladite étape a) est inférieure à 99%.

2. Procédé selon la revendication 1 dans lequel ladite charge comprend de 40 à 100% poids d'au moins un monoalcool primaire.

3. Procédé selon l'une des revendications précédentes dans lequel ledit monoalcool primaire est l'isobutanol ou le 2-methyl-1-butanol, pris seul ou en mélange.

4. Procédé selon l'une des revendications précédentes dans lequel ladite zéolithe contient également au moins une série de canaux dont l'ouverture de pores est définie par un anneau contenant 10 atomes d'oxygène (10 MR).

5. Procédé selon la revendication précédente dans lequel ladite zéolithe est choisie parmi les zéolithes de type structural FER et MFS, prises seules ou en mélange.

6. Procédé selon la revendication précédente dans lequel ladite zéolithe est choisie dans le groupe constitué par, pour le type FER, les zéolithes ferrierite, FU-9, ISI-6, NU-23, ZSM-35 et pour le type MFS, la zéolite ZSM-57, et leurs mélanges.

**7.** Procédé selon l'une des revendications précédentes dans lequel la durée d'arrêt de ladite étape b) est comprise entre 0,5 et 3 h.

**8.** Procédé selon l'une des revendications précédentes dans lequel la durée d'arrêt de ladite étape b) est comprise entre 0,5 et 2 h.

**9.** Procédé selon l'une des revendications précédentes dans lequel ladite étape de sélectivation comprend l'arrêt de production d'effluent de ladite étape réactionnelle a).

**Patentansprüche**

**1.** Verfahren zur Dehydratisierung von Alkoholen zu Olefinen, umfassend:

a) einen Reaktionsschritt, dem eine Ladung zugeführt wird, die mindestens einen primären Monoalkohol mit der Formel $R-CH_2-OH$ umfasst, in der R ein nicht geradkettiger Alkylrest mit der allgemeinen Formel $C_nH_{2n+1}$ ist, worin n eine ganze Zahl zwischen 3 und 20 ist, wobei der Reaktionsschritt in der Gasphase bei einer gewichteten mittleren Temperatur zwischen 250 und 400 °C, bei einem Druck zwischen 0,2 MPa und 1 MPa und bei einer WHSV zwischen 1 und 18 $h^{-1}$ in Gegenwart eines Katalysators vorgenommen wird, der einen Zeolithen umfasst, der mindestens eine Reihe von Kanälen umfasst, deren Öffnung durch einen Ring mit 8 Sauerstoffatomen (8 MR) definiert ist, der einen olefinischen Abstrom erzeugt;
b) einen Schritt zur Selektivierung des Katalysators, der, während einer Dauer zwischen 0,5 und 5 h, das Stoppen der Zuführung und das Halten der Temperatur und des Drucks des Schritts a) umfasst;
c) das Wiederaufnehmen der Zuführung nach dem Schritt b) mit Rückkehr zu den Bedingungen des Schritts a);

wobei der Selektivierungsschritt umgesetzt wird, wenn die Selektivität an Iso-olefinen **n** des Schritts a) mehr als 25 % beträgt oder wenn die Umwandlung zu Alkoholen des Schritts a) weniger als 99 % beträgt.

**2.** Verfahren nach Anspruch 1, bei dem die Ladung 40 bis 100 Gew.-% mindestens einen primären Monoalkohols umfasst.

**3.** Verfahren nach einem der vorhergehenden Ansprüche, bei dem der primäre Monoalkohol Isobutanol oder 2-Methyl-1-butanol ist, allein oder im Gemisch.

**4.** Verfahren nach einem der vorhergehenden Ansprüche, bei dem der Zeolith auch mindestens eine Reihe von Kanälen enthält, deren Porenöffnung durch einen Ring definiert ist, der 10 Sauerstoffatome enthält (10 MR).

**5.** Verfahren nach dem vorhergehenden Anspruch, bei dem der Zeolith unter den Zeolithen vom Strukturtyp FER und MFS gewählt ist, allein oder im Gemisch.

**6.** Verfahren nach dem vorhergehenden Anspruch, bei dem der Zeolith aus der Gruppe gewählt ist, die für den FER-Typ aus den Zeolithen Ferrierit, FU-9, ISI-6, NU-23, ZSM-35 und für den MFS-Typ aus dem Zeolith ZSM-57 und ihren Gemischen besteht.

**7.** Verfahren nach einem der vorhergehenden Ansprüche, bei dem die Dauer des Stoppens des Schritts b) zwischen 0,5 und 3 h beträgt.

**8.** Verfahren nach einem der vorhergehenden Ansprüche, bei dem die Dauer des Stoppens des Schritts b) zwischen 0,5 und 2 h beträgt.

**9.** Verfahren nach einem der vorhergehenden Ansprüche, bei dem der Selektivierungsschritt das Stoppen der Erzeugung von Abstrom des Reaktionsschritts a) umfasst.

**Claims**

**1.** Process for dehydrating alcohols to olefins, comprising:

a) a reaction step fed with a feedstock comprising at least one primary monoalcohol of formula R-CH$_2$-OH, in which R is a non-linear alkyl radical of general formula C$_n$H2$_{n+1}$ where n is an integer between 3 and 20, said reaction step being performed in the gas phase at a weighted average temperature of between 250 and 400°C, at a pressure of between 0.2 MPa and 1 MPa and at a WWH of between 1 and 18 h$^{-1}$ in the presence of a catalyst comprising a zeolite comprising at least one series of channels whose opening is defined by a ring of 8 oxygen atoms (8MR) producing an olefinic effluent;

b) a catalyst selectivation step comprising, for a period of between 0.5 and 5 hours, stoppage of the feed and maintenance of the temperature and pressure of said step a);

c) resumption of the feed on conclusion of step b) with return to the conditions of step a);

said selectivation step being performed when the selectivity towards isoolefins n of said step a) is greater than 25%, or when the conversion into alcohols of said step a) is less than 99%.

2. Process according to Claim 1, in which said feedstock comprises from 40% to 100% by weight of at least one primary monoalcohol.

3. Process according to either of the preceding claims, in which said primary monoalcohol is isobutanol or 2-methyl-1-butanol, taken alone or as a mixture.

4. Process according to one of the preceding claims, in which said zeolite also contains at least one series of channels, the pore opening of which is defined by a ring containing 10 oxygen atoms (10 MR).

5. Process according to the preceding claim, in which said zeolite is chosen from zeolites of the FER and MFS framework types, taken alone or as a mixture.

6. Process according to the preceding claim, in which said zeolite is chosen from the group consisting, for the FER type, of ferrierite, FU-9, ISI-6, NU-23 and ZSM-35 zeolites, and, for the MFS type, of ZSM-57 zeolite, and mixtures thereof.

7. Process according to one of the preceding claims, in which the period of stoppage of said step b) is between 0.5 and 3 hours.

8. Process according to one of the preceding claims, in which the period of stoppage of said step b) is between 0.5 and 2 hours.

9. Process according to one of the preceding claims, in which said selectivation step comprises the stoppage of effluent production of said reaction step a).

**Figure 1 :** Profils thermiques dans le lit catalytique mesurés avant, pendant et après l'étape de sélectivation de 70 minutes.

**Figure 2 :** Profils thermiques dans le lit catalytique mesurés avant, pendant, et après l'étape de sélectivation de 25 minutes.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2348005 A
- WO 2011113834 A
- EP 3040125 A

**Littérature non-brevet citée dans la description**

- **BENITO et al.** *Ind.Eng.Chem.Res.,* 1996, vol. 35, 2177-2182
- **CH. BAERLOCHER ; L. B. MC CUSKER ; D.H. OISON.** Atlas of Zeolite Structure Types. Elsevier, 2007